# EUROPEAN PATENT SPECIFICATION

(11) **EP 0 237 269 B1**
(45) Date of publication and mention of the grant of the patent: **28.04.1993**
(21) Application number: 87301908.7
(22) Date of filing: 05.03.1987
(51) Int. Cl.: C12N 15/00, C12N 15/69, C12N 1/20

(54) **Replication control mutants**
Mutanten zur Replikationskontrolle
Mutants à réplication contrôlée

(30) Priority: 14.03.1986 GB 8606384
(43) Date of publication of application: 16.09.1987
(73) Proprietor: IMPERIAL CHEMICAL INDUSTRIES PLC, London SW1P 3JF (GB)
(72) Inventor: Barth, Peter Thomas, Helsby Cheshire WA6 9PX (GB)
(74) Representative: Thomas, Ieuan

(56) References cited:
- WO-A-79/00467
- JOURNAL OF BACTERIOLOGY, vol. 150, no. 2, May 1982, pages 552-562, Washington, DC, US; R. MEYER et al.: "Properties of R1162, a broad-host-range, high-copy-number plasmid"
- BASIC LIFE SCIENCE, vol. 30, 1985, pages 173-188, New York, US; R.J. MEYER et al.: "Broad host-range plasmid R1162: replication, incompatibility, and copy-number control"
- Proc. Natl. Acad. Sci. USA, vol. 82, p. 6090 (Sept. 1985)

## Description

This invention relates to plasmid vectors of variable copy number and particularly to the temperature control of such copy number. Such plasmid vectors are useful in the preparation of recombinant DNA molecules and in the control of the expression of genes cloned therein.

In gene cloning, it is often desirable to obtain from a bacterial culture of a strain, the maximum possible yield of the protein expressed by a gene which has been introduced into the strain. Typically, plasmids are used as vectors for effecting this introduction.

It is known that where the number of copies of a certain gene within a bacterial host cell, i.e. so-called "gene dosage", is increased the yield of the protein encoded by that gene is increased. However, many proteins, where they are expressed above a certain level, are lethal to their bacterial hosts. For example, certain, even apparently benign, proteins may become lethal where they appropriate too great a proportion of the host cell's natural protein production. It would, therefore, be desirable to control the gene dosage by some convenient parameter such that (a) under low expression conditions, genes could be controlled and cultures grown without undue detriment to the host strain and (b) subsequently, high expression conditions could be imposed such that a higher level of expression, e.g. the maximum sustainable yield of the desired protein, could be obtained from such cultures.

Temperature variation, we believe, appears to be a suitable procedure for varying the gene dosage of cloned genes, particularly by varying the copy number of the plasmid vector with the temeprature at which the host is grown. Alternative procedures for varying the copy number are known in the art, for example, the "amplification" of the vector pBR 322 by the addition of a certain antibiotic, i.e. streptomycin. However, this procedure blocks protein synthesis and, accordingly, such a procedure would not be suitable for the expression of cloned genes. Furthermore, addition of an antibiotic is often undesirable in the products, particularly those which are destined for medical applications. The aforesaid temperature variation has the further advantage that it may also permit more than one expression-controlling mechanism to be adjusted simultaneously, thus tending to afford greater control.

It will be appreciated that plasmids with a broad host range tend to be more useful than the narrow host-range plasmids often presently used in gene cloning. Such broad-host range plamids often afford the possibility of choosing the optimal host for the production of a particular protein from a broader range of hosts.

We have now devised broad host range plasmids which (a) have high copy numbers and (b) have temperature sensitive copy number control, i.e. replication occurs at high copy number at high temperatures but at lower copy number at lower temperatures.

By "broad host range plasmids" we mean plasmids which can be introduced into and stably maintained in a broad range of bacteria. Broad host range plasmids tend to have the advantage that the optimal host for production of a particular protein can be chosen from a broader range.

According to a first aspect of the present invention there is provided a plasmid vector which is derivable by mutation of a broad host range plasmid such that it exists as a higher copy number mutant thereof.

Preferably the broad host range plasmids from which plasmid vectors according to the present invention are derivable belong to the incompatability group Q (IncQ) and more preferably it is R300B (Barth and Grinter, J. Bacteriol., 1974, 120, 610-630), which is the prototype of the Inc Q group. Plasmid R300B is known to be relatively small (8.7 kb), to have a copy number of about 30 per cell in E. coli, to be readily mobilised into a broad range of Gram-negative bacteria and to be stably maintained in many Gram-negative bacterial species (Barth, Tobin and Sharpe, 1981, from "Molecular Biology Pathogenicity and Ecology of Bacterial Plasmids", eds. Levy, Clowes and Koenig, Plenum Publishing corporation). Furthermore, we have described the use of inter alia Inc Q group plasmids as vectors, e.g. in European Patent Specification No 37273B.

The aforesaid mutation may be effected by physical means, for example by electromagnetic radiation, e.g. U.V. light; or preferably by chemical means, e.g. with hydroxylamine, or more preferably with ethyl methanesulphonate or N-methyl-N'nitro-N-nitrosoguanidine. The treatment with N-methyl-N'-nitro-N-nitroguanidine can be conveniently effected as described by Ornston (Journal of Biological Chemistry, 1966, 241, pages 3800-3810).

Such mutants often have a raised plasmid copy number and a few were found to be temperature-sensitive for plasmid copy number control (which temperature-sensitive property is hereinafter referred to for convenience as "cop^{ts}"; and which mutants having such property are hereinafter referred to for convenience as "cop^{ts} mutants"). The increase in copy number at high temperature has tended to be small, e.g. about 3-fold, on raising the temperture from about 30°C to about 42°C.

Further mutation of the aforesaid cop^{ts} mutants, under mutation conditions as hereinbefore described, often gives second mutants with a larger cop^{ts} response, e.g. pTB233. For example, one second mutant (pTB 225) has a high constitutive copy number (about 30-fold higher that its parent cop^{ts} mutant) but significantly depresses the growth rate of its host, and is not a "cop^{ts}" mutant.

We have now found that, by adjusting the temperature at which certain of the aforesaid cop^{ts} mutants are used, appropriately desirable copy numbers of the vector may be obtained such that expression of a protein, which is not lethal to the host and which is encoded by a gene incorportated in the vector, may be obtained at a maximum sustainable field.

According to a second aspect of the present invention there are provided broad host range plasmid vectors which are temperature sensitive for plasmid copy number control.

Nordström et al have shown that plasmid copy number mutants of certain narrow host range plasmids can be selected by using resistance to ampicillin conferred by the bla gene. There appears to be a linear relationship between the copy number of the bla gene and the level of ampicillin resistance conferred by the plasmid on the host microorganism into which it has been introduced.

The two resistance-genes already present in, for example R300 B, i.e. sul and aphC, which confer resistance to sulphonamides and streptomycin respectively, do not have this advantage. Furthermore, sul resistance is at a very high level in strains bearing R300b and cannot be significantly increased with an increase in sul gene dosage; the aphC resistance can be increased slightly but reaches a plateau too soon to be of substantial commercial use

We have found that Nordström's technique can conveniently be used to select plasmid vectors according to the first aspect of the present invention although we do not exclude the possibility that they may be selected by alternative procedures known to the skilled man or to be developed.

In the preparation of a vector according to the first aspect of the present invention using Nordstom's procedure, the bla gene is inserted into a site on the broad host range vector,e.g. R300B such that it does not unduly impair or destroy, and preferably does not impair in any way, the useful properties of the vector, particulary the stable maintenance in a broad range of bacterial hosts. It is preferably inserted at the Eco RI restriction site between the sul and aphc antibiotic resistance genes of R300B in what appears to be a so-called genetically silent region. The plasmid produced thereby, hereinafter referred to for convenience as pTB200, retains substantially all the useful properties of R300B. Additionally, the presence of the bla gene in modified vectors, e.g. pTB200, permits the selection of mutant plasmids which are resistant to high levels of ampicillin in the presence of clavulanic acid; at least the majority of such mutants have an increased copy number.

Where a bla gene is used in our aforesaid procedure it is preferably isolated from plasmid pBR322, although we do not exclude the possibility that it may be isolated from any other suitable plasmid, e.g. R1, R4, etc.

To facilitate the cloning of foreign genes into plasmid vectors according to the present invention it is preferred that a DNA sequence having mulitple restriction sites, e.g. from phage M13 mp18, or plasmid puc 18, inserted upstream of the bla gene where it is present.

Such multi-cloning sequences may be inserted into plasmids according to the present invention, e.g. pTB200 or pTB225, in a two step process. The plasmids are restricted partially with Aat II and then ligated with a synthetic oligomer that converts this site to one recognising Hind III. There are three Aat II sites in the vectors and each of these may be converted; preferably, however, the site closest to the Eco R1 site is used. This site conversion has been found in inter alia pTB200 and pTB 225. In the second step, the Eco RI to Hind III multicloning site sequence is inserted between the Eco RI and Hind III sites created in the first step thus giving vectors pTB 244 and 254 respectively. The cop^{ts} mutant pTB 233 also has the multicloning site sequence introduced, on a restriction fragment taken from pTB244, to give pTB234. The vectors pTC244, 245 and 234 retain the properties of their parents pTB8200, 225 and 233. Foreign genes cloned into the multicloning site in any of these vectors will be under the transcriptional control of the strong upstream promotor that expresses sul.

The location of cop mutations in certain mutant plasmids has been deduced from restriction fragment swap experiments. We have found that the original cop^{ts} mutation in, for example, pTB220 is found in the 2.7-4.7 kb co-ordinates region. This region contains the oriV replication origin site, which is probably affected by the cop^{ts} mutation. (We have found that the secondary nmutation, leading to the very high copy number of pTB225, is located in the 6.6-8.6kb co-ordinates region. That region contains the repC gene, whic is known to be involved in replication control (Haring, Scholz, Scherzinger, Frey, Derbyshire, Hatfull, Willetts and Bagdaserian, Proc. Natl. Acad.Sci., 1985, 82, 6090-6094).

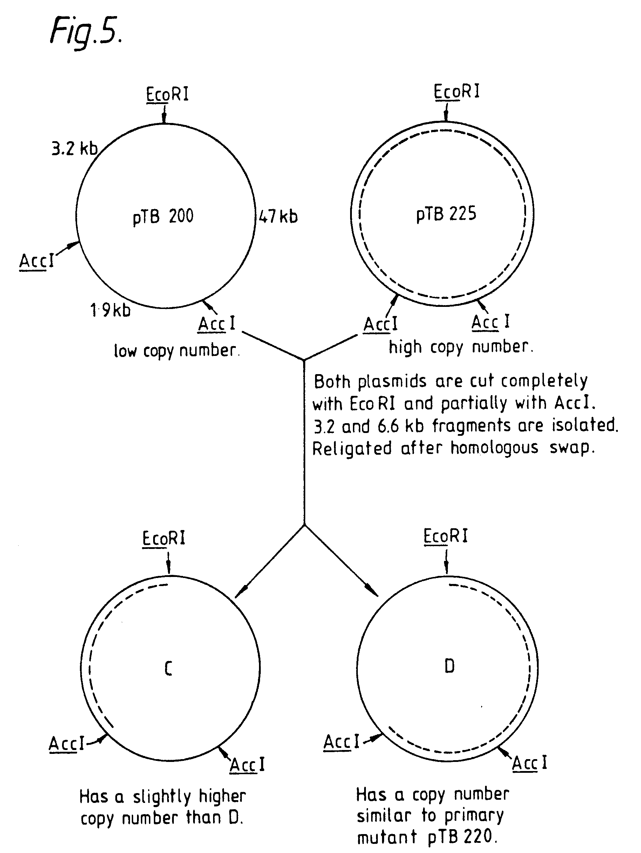

As can be seen from Figure 5, neither mutation alone has a large effect on copy number, in combination they exhibit synergism to cause the impressive phenotype of pTB225. Accordingly, it is preferred that plasmids according to the present invention are derivable from plasmids by effecting the first and second mutations on opposite sides of the broad host range plasmid.

It is believed that mutations near the origin of replication of plasmids according to the second aspect of the present invention provide for temperature regulation of the copy number thereof.

As examples of genes whch may be present in plasmid vectors according to the present invention may be mentioned inter alia Bla and Cat,

Firstly, with respect to the constitutively high copy number vectors pTB225 and 254: whereas the parent pTB200 gave 36.3 units/mg protein of β -lactam, pTB225 gave 1033.6 units/mg protein under the same growth conditions, at 37°C, i.e. an increase of more than 28 fold. After cloning in the cat gene pTB244:: cat gave 0.29 units/mg protein of the CAT activity whereas pTB254::cat gave 8.34 units/mg protein, which is a similar increase ratio. With respect to the cop^{ts} vector pTB234, the β -lactamase activities at 30° and 42° were 33.6 and 412 units/mg protein respectively and after introduction of the cat gene, the CAT activities were 0.53 and 8,84 units/mg protein respectively. This demonstrateS that the expression of both genes responds to the copy number change of the vector with growth temperature.

The present invention is further illustrated by, but not limited to, the following examples.

### EXPERIMENT A

This Experiment describes the preparation of a plasmid which is useful in the preparation of plasmids according to the present invention.

By procedures well known in the art, pBR322 was cut with EcoRI and partially with AhaIII and a fragment of 1131bp was isolated; R300B was cut with EcoRI and HpaI and the aforesaid fragment was cloned thereinto. The product plasmid pTB200 contained the bla gene inserted between the antibiotic resistance genes (sul and aphC) and having the same orientation of transcription. pTB200 was found to have suffered no loss of function and to retain the copy number, stability, mobilizability and host-range of its parent R300B.

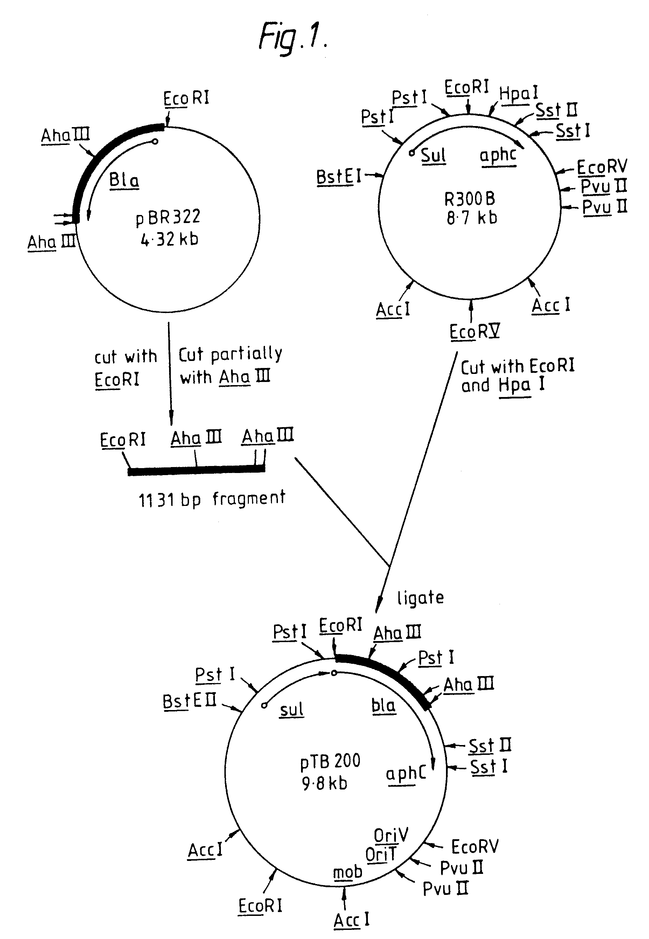

The preparation of pTB200 is shown in outline in Figure 1.

### EXAMPLE 1

This Example illustrates the preparation of vector plasmids according to the first aspect of the present invention.

We have found that the plasmid pTB200 described in Experiment A confers a resistance to ampicillin (Ap^{R}) of about 1000 microgram/ml in E.coli and that selection to higher levels is difficult because of solubility limitations. However, the addition of clavulanic acid at 1 microgram/ml brings Ap^{R} down to 20 microgram/ml (minimal inhibitory concentration). Cultures of C600 (pTB200) were mutagenised with a variety of mutagens, particularly with N-nitrosoguanidine under conditions well known in the art, plasmid DNA was isolated and transformed into C600. Transformants were selected at 50-100 microgram/ml of ampicillin (plus clavulanic acid at 1 microgram/ml) at 42°C.

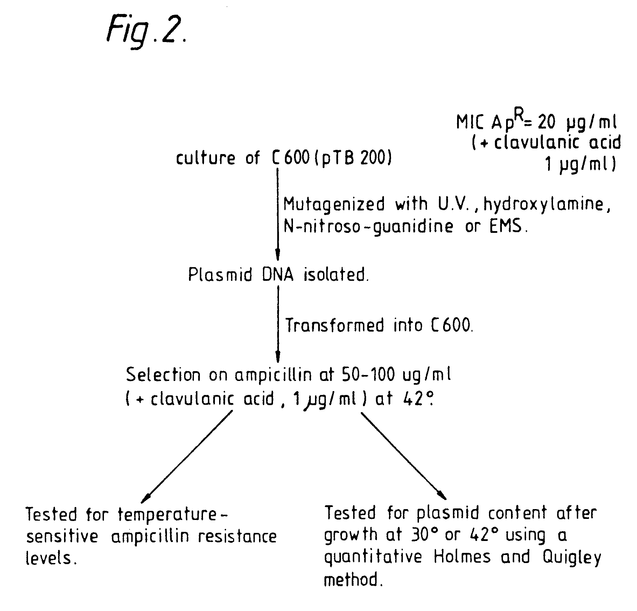

The mutants were screened using the method of plasmid isolation described by Holmes and Quigley. Cultures were grown overnight at 30° and 42°C and constant cell cultures used in a highly reproducible technique. The aforesaid screening and isolation is shown diagrammatically in Figure 2.

From about 10⁶ potential transformants, 430 with a high Ap^{R} at 42°C were isolated and screened. Most were constitutive copy number mutants but only three (cop^{ts} mutants) had a significant temperature sensitive phenotype (pTB210, pTB220 and pTB230).

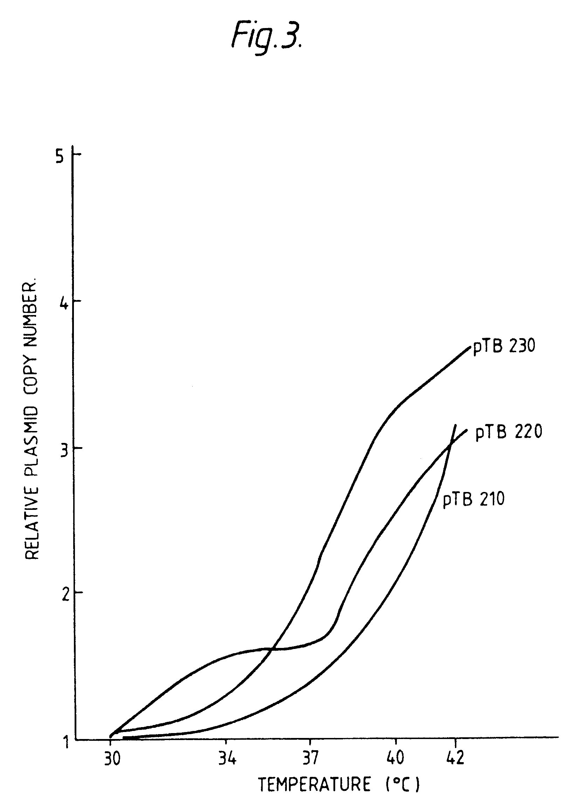

The relative copy number response of the aforesaid three cop^{ts} mutants to growth of their cultures at various temperatures are shown in Figure 3. These results were obtained by direct Holmes and Quigley plasmid analysis.

The increase in copy number, although significant, was about 3-fold from 30°C to 42°C for each of the three mutants.

### EXAMPLE 2

This example illustrates the isolation of secondary mutants from cop^{ts} mutants.

The three cop^{ts} mutants prepared in Example 1 were subjected to further mutagenesis under the conditions described in Example 1. Transformants were selected on higher levels of ampicillin (125-175 microgram/ml with clavulanic acid, 1 microgram/ml) at 42°C. About 150 secondary mutants were isolated and analysed.

One of the secondary mutants (hereinafter referred to for convenience as pTB225) gave a very high constitutive copy number, estimated to be about 30-fold greater than its grand-parent (pTB200), i.e. this plasmid exists at about 1000 copies per cell. It depresses the growth rate of its host significantly but is not lethal.

### EXAMPLE 3

This Example illustrates the introduction of M13mp18 multicloning sites into pTB200 and 225.

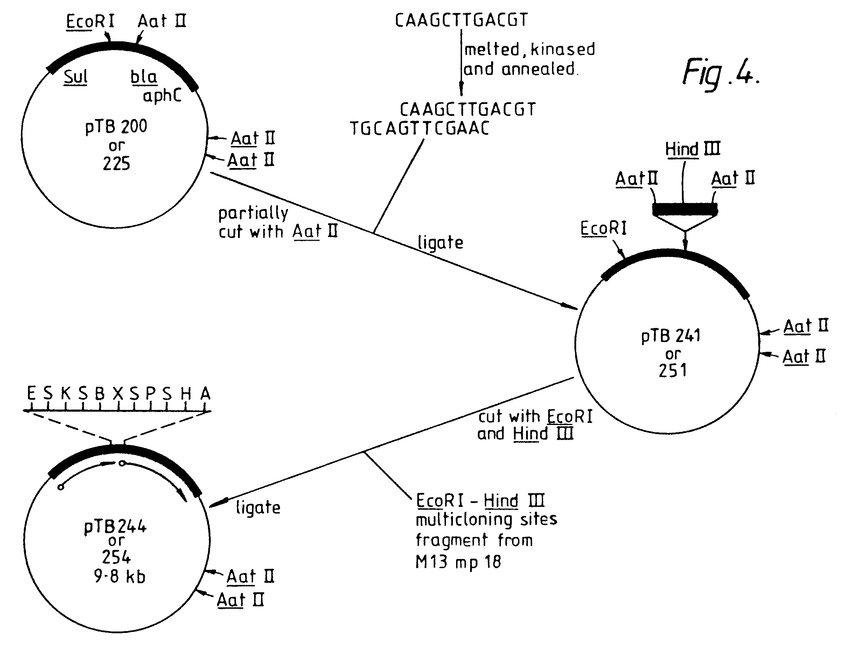

A synthetic oligomer having the DNA sequence "CAAGCTTGACGT", synthesised by procedures well known in the art, was melted, kinased and annealed and then was inserted into pTB200 and pTB225 after the plasmids had been partially cut with AatII. Progeny plasmids were analysed for those that had acquired the insert close to the EcoRI restriction site (i.e. pTB241, low copy number; pTB251 high copy number). The unique Hind III restriction site which had been introduced thereby was then used to insert the EcoRI to HindIII DNA multicloning site sequence from M13mp18; pTB244 and pTB254 were prepared. This two-stage procedure is shown in outline in Figure 4.

Thus, broad host range vectors with low and high copy numbers containing useful multicloning sites, most of which remain unique, under the transcriptional control of the powerful sul promoter have been prepared.

Secondary cop^{ts} mutants have also had the multicloning sites introduced to produce, e.g. pTB234. These should be useful for the cloning of foreign genes and their conditional expression under temperature control.

An outline of the derivation of certain of the plasmid vectors according to the present invention is as follows:

R300B    naturally occuring plasmid,
broad host range, Inc Q

bla gene isolated from pBR322

pTB200
pTB201, 202, 203 etc
constitutive higher
copy no. mutants
mutagenesis, selection on high
Ap levels and plasmid content
screening

pTB210, 220, 230  copts mutants

secondary
mutation,
selection
and screening

pTB225
constitutive,
very high copy
number mutant
pTB233
enhanced copts
mutant

Insertion of the
multicloning site
sequence from M13mp18

pTB244    pTB254    pTB234

## Claims

1. A broad host range plasmid vector which can be introduced into and stably maintained in a broad range of bacteria, which vector is derivable by mutation of a broad host range plasmid of the incompatability group Q such that it exists in a higher copy number mutant thereof and is temperature sensitive for plasmid copy number control.

2. A plasmid vector as claimed in claim 1 wherein the broad host range plasmid is R300B.

3. A plasmid vector as claimed in claim 1 wherein the mutation is effected by chemical means.

4. A plasmid vector as claimed in claim 3 wherein the chemical means comprises treatment with ethyl methanesulphonate or N-methyl-N'-nitro-N-nitrosoguanidine.

5. A plasmid vector which is derivable by mutation of a plasmid vector as claimed in claim 1 or 2 and which is temperature sensitive for plasmid copy number control.

6. A plasmid vector as claimed in claim 1 or 5 comprising a DNA sequence having multiple cloning sites.

7. A plasmid vector as claimed in claim 1 wherein the cop^{ts} mutation is located in the 2.7-4.7kb co-ordinates region.

8. A plasmid vector as claimed in claim 7 wherein a further mutation is located in the 6.6-8.6kb co-ordinates region.

9. A recombinant DNA molecule derived from a plasmid vector as claimed in claim 1.

10. A Gram-negative bacterial cell comprising a recombinant DNA molecule as claimed in claim 9.

11. A process for the preparation of a plasmid vector as claimed in claim 1 which process comprises the step of selection using the bla gene.

12. A process as claimed in claim 11 which process comprises the step of inserting the bla gene into a site on the broad host range plasmid such that it does not unduly impair or destroy the useful properties thereof.

13. A process as claimed in claim 12 which process comprises the steps of converting a Aat II site into a Hind III site.

14. A process as claimed in claim 13 wherein the Aat II near the EcoRI site is converted.

15. A process as claimed in claim 11 for the preparation of a plasmid vector as claimed in claim 8 wherein the first and second mutations are effected on opposite sides of the broad host range plasmid.

## Patentansprüche

1. Plasmidvector mit breitem Wirtsbereich, der in einen breiten Bereich von Bakterien eingeführt und stabil beibehalten werden kann, wobei der Vector durch Muatation eines Plasmids mit breitem Wirtsbereich der Incompatabilitätsgruppe Q erhalten werden kann, und zwar derart, daß er als Mutante davon mit höherer Kopienzahl vorliegt und zur Steuerung der Plasmidkopienzahl temperaturempfindlich ist.

2. Plasmidvector nach Anspruch 1, wobei es sich bei dem Plasmid mit breitem Wirtsbereich um R300B handelt.

3. Plasmidvector nach Anspruch 1, wobei die Mutation durch chemische Mittel bewirkt wird.

4. Plasmidvector nach Anspruch 3, wobei das chemische Mittel die Behandlung mit Ethylmethansulfonat oder N-Methyl-N'-nitro-N-nitrosoguanidin umfaßt.

5. Plasmidvector, der durch Mutation eines Plasmidvectors nach den Ansprüchen 1 oder 2 erhältlich ist und zur Steuerung der Plasmidkopienzahl temperaturempfindlich ist.

6. Plasmidvector nach den Ansprüchen 1 oder 5, der eine DNA-Sequenz mit mehreren Clonierstellen aufweist.

7. Plasmidvector nach Anspruch 1, wobei die Cop^{ts}-Mutation in der 2,7-4,7 kb Koordinatenregion lokalisiert ist.

8. Plasmidvector nach Anspruch 7, wobei eine weitere Mutation in der 6,6-8,6 kb Koordinatenregion lokalisiert ist.

9. Recombinantes DNA-Molekül, das von einem Plasmidvector nach Anspruch 1 abgeleitet ist.

10. Gram-negative Bakterienzelle, die ein recombinantes DNA-Molekül nach Anspruch 9 enthält.

11. Verfahren zur Herstellung eines Plasmidvectors nach Anspruch 1, wobei das Verfahren den Selectionsschritt unter Verwendung des bla-Gens aufweist.

12. Verfahren nach Anspruch 11, wobei das Verfahren den Insertionsschritt des bla-Gens in eine Stelle auf dem Plasmid mit breitem Wirtsbereich aufweist, und zwar derart, daß dessen nützliche Eigenschaften nicht unnötig beeinträchtigt oder zerstört werden.

13. Verfahren nach Anspruch 12, wobei das Verfahren die Konvertierungsschritte einer Aat II-Stelle in eine Hind III-Stelle aufweist.

14. Verfahren nach Anspruch 13, wobei die Aat II-Stelle in der Nähe der EcoRI-Stelle convertiert wird.

15. Verfahren nach Anspruch 11 zur Herstellung eines Plasmidvectors nach Anspruch 8, wobei die erste und zweite Mutation an entgegengesetzten Stellen auf den Plasmid mit breitem Wirtsbereich bewirkt wird.

## Revendications

1. Vecteur consistant en un plasmide à large gamme d'hôtes, qui peut être introduit et maintenu de manière stable dans une large gamme de bactéries, vecteur qui peut être obtenu par mutation d'un plasmide, à large gamme d'hôtes, du groupe d'incompatibilité Q de telle sorte qu'il existe sous forme d'un de ses mutants à plus grand nombre de copies et qu'il soit sensible à la température pour la régulation du nombre de copies de plasmide.

2. Vecteur consistant en un plasmide suivant la revendication 1, caractérisé en ce que le plasmide à large gamme d'hôtes est le plasmide R300B.

3. Vecteur consistant en un plasmide suivant la revendication 1, caractérisé en ce que la mutation est provoquée par des moyens chimiques.

4. Vecteur consistant en un plasmide suivant la revendication 3, caractérisé en ce que les moyens chimiques consistent en un traitement avec le méthanesulfonate d'éthyle ou la N-méthyl-N'-nitro-N-nitrosoguanidine.

5. Vecteur consistant en un plasmide qui peut être produit par mutation d'un vecteur consistant en un plasmide suivant la revendication 1 ou 2 et qui est sensible à la température pour la régulation du nombre de copies de plasmide.

6. Vecteur consistant en un plasmid e suivant la revendication 1 ou 5, comprenant une séquence d'ADN ayant des sites de clonage multiples.

7. Vecteur consistant en un plasmide suivant la revendication 1, dans lequel la mutation cop^{ts} est située dans la région de coordonnées 2,7-4,7 kb.

8. Vecteur consistant en un plasmide suivant la revendication 7, dans lequel une mutation supplémentaire est située dans la région de coordonnées 6,6-8,6 kb.

9. Molécule d'ADN recombinant dérivée d'un vecteur consistant en un plasmide suivant la revendication 1.

10. Cellules bactérienne Gram-négative comprenant une molécule d'ADN recombinant suivant la revendication 9.

11. Procédé de préparation d'un vecteur consistant en un plasmide suivant la revendication 1, qui comprend l'étape de sélection au moyen du gène bla.

12. Procédé suivant la revendication 11, qui comprend l'étape d'insertion du gène bla dans un site sur le plasmide à large gamme d'hôtes de telle sorte qu'il n'entrave pas excessivement ou ne détruise pas ses propriétés utiles.

13. Procédé suivant la revendication 12, qui comprend les étapes de conversion d'un site Aat II en un site Hind III.

14. Procédé suivant la revendication 13, dans lequel le site Aat II à proximité du site EcoRI est soumis à la conversion.

15. Procédé suivant la revendication 11 pour la préparation d'un vecteur consistant en un plasmide suivant la revendication 8, dans lequel les première et seconde mutations sont provoquées sur des côtés opposés du plasmide à large gamme d'hôtes.
